(19) **Europäisches Patentamt / European Patent Office / Office européen des brevets**

(11) **EP 2 246 094 B1**

(12) **FASCICULE DE BREVET EUROPEEN**

(45) Date de publication et mention
de la délivrance du brevet:
**09.03.2011  Bulletin 2011/10**

(51) Int Cl.:
***A61N 1/378*** *(2006.01)*

(21) Numéro de dépôt: **10155583.7**

(22) Date de dépôt: **05.03.2010**

(54) **Alimentation à découpage inductive à contrôle numérique pour dispositif médical implantable actif**

Induktives Schaltnetzteil mit digitaler Steuerung für aktive implantierbare medizinische Vorrichtung

Digitally controlled power supply with inductive switching for an active implantable medical device

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **28.04.2009  FR 0952775**

(43) Date de publication de la demande:
**03.11.2010  Bulletin 2010/44**

(73) Titulaire: **Sorin CRM SAS**
**92541 Montrouge (FR)**

(72) Inventeur: **Pons, Pascal**
**38500, LA BUISSE (FR)**

(74) Mandataire: **Dupuis-Latour, Dominique**
**SEP Bardehle Pagenberg Dost Altenburg Geissler**
**10, boulevard Haussmann**
**75009 Paris (FR)**

(56) Documents cités:
EP-A- 0 719 570          EP-A- 1 647 303
DE-A1-102006 008 495     US-A- 5 769 877
US-A1- 2004 082 982      US-A1- 2008 077 010
US-A1- 2009 043 244

**Description**

L'invention concerne les dispositifs médicaux actifs, notamment les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation, de cardioversion et/ou de défibrillation en cas de trouble du rythme détecté par le dispositif. L'alimentation en énergie est un aspect particulièrement critique de ces dispositifs, en raison des contraintes très strictes de miniaturisation et de durée de vie de la batterie.

Les documents US 5 769 877 A, EP 0 719 570 A1, EP 1 647 303 A1, DE 10 2006 008 495 A1, US 2008/0077010 A1, US 2009/0043244 A1 et US 2004/00892982 A1 décrivent divers aspects de l'alimentation en énergie d'implants médicaux, concernant respectivement

- l'alimentation transcutanée d'un implant, le stockage de l'énergie dans un condensateur et la régulation de tension par un circuit linéaire;
- un translateur de tensions pour stimulateur implanté, fonctionnant avec des circuits de type "pompe à charges" utilisant des commutateurs séquencés reliés à différents niveaux de tension;
- l'alimentation d'un circuit de télémétrie RF via un accumulateur chargé par un multiplicateur de tension à commmutation de capacités;
- un actionneur contrôlé par un détecteur d'activité neurologique du cerveau;
- l'évaluation du pouls d'un patient par une technique Doppler à ultrasons;
- une pompe de délivrance de médicament asservie aux variations de la résistance corporelle; et
- un oscillateur à quartz à faible consommation, mettant en oeuvre un multiplicateur de tension à commutation de capacités.

En particulier, un implant actif tel qu'un stimulateur ou défibrillateur implantable est pourvu d'une batterie dont la durée de vie nominale est de l'ordre d'une dizaine d'années. Au cours de son utilisation, cette batterie présente des variations notables de tension à ses bornes :

- sur le très long terme : ainsi, entre le moment de l'implantation et la fin de vie de la batterie, la tension moyenne diminue progressivement de 3,3 à 2,4 V, soit une baisse de plus de 25 % ;
- mais également pendant des périodes très courtes: sous l'effet de brusques appels de courant, la tension de la batterie chute du fait de sa résistance interne propre.

Concernant plus précisément ce second point, alors que la consommation moyenne d'un stimulateur en temps normal est de l'ordre de 15 µA, dans certains cas peuvent survenir de brusques appels de courant, notamment lorsque des fonctions de télémétrie RF (radiofréquence) sont activées, l'alimentation de l'émetteur/récepteur nécessitant un courant de l'ordre de 5 à 10 mA.

Bien plus, dans le cas d'un défibrillateur implantable, après délivrance d'un choc la recharge du condensateur haute tension peut créer un appel temporaire de courant pouvant atteindre 3 à 4 A.

De ce fait, dans les cas extrêmes, la tension aux bornes de la batterie peut chuter jusqu'a des tensions aussi basses que 1,2 V, soit une chute de tension de plus de 50 % en l'espace de quelques dizaines ou centaines de microsecondes.

Ces variations très importantes, à très long terme ou quasi-instantanées, de la tension d'alimentation imposent l'utilisation de circuits d'alimentation spécifiques permettant de garantir aux divers organes du dispositif la délivrance d'une tension précise, parfaitement stabilisée.

Les circuits d'alimentation utilisés à cette fin sont du type convertisseur à découpage ou SMPS *(Switching Mode Power Supply).*

Le principe de ces circuits consiste à opérer de manière cyclique, avec une phase primaire lors de laquelle une inductance est chargée en énergie à partir de la tension délivrée par la batterie, puis une phase secondaire lors de laquelle cette inductance est déchargée vers le circuit utilisateur. Des condensateurs tampons disposés en entrée et en sortie permettent d'assurer une certaine constance de la tension délivrée, dont la valeur est régulée en faisant varier les durées des phases de charge et de décharge et/ou le rapport cyclique de ces durées.

Il existe plusieurs topologies de convertisseurs à découpage, choisies en fonction des besoins, notamment les topologies dites *buck* (avec abaissement de la tension), *boost* (avec élévation de la tension), *buck-boost* (avec abaissement ou élévation de la tension, et inversion de polarité), *mixed* (avec abaissement ou élévation de la tension, et sans inversion de polarité).

D'autre part, avec une même batterie il est possible d'associer plusieurs alimentations dédiées à l'alimentation de circuits différents, ces alimentations partageant une même inductance.

Le contrôle des phases de charge et de décharge s'effectue en général par mesure du courant traversant l'inductance. Celle-ci est alimentée avec un courant qui va croissant, jusqu'à atteindre une valeur prédéterminée (ci-après "courant de crête"), cette valeur étant par exemple fonction du signal d'erreur mesuré entre la tension de sortie du convertisseur et la tension nominale devant être délivrée au circuit à alimenter. Lorsque le courant de crête est atteint, la charge est interrompue et l'inductance est alors déchargée vers le circuit utilisateur.

Les phases de charge/décharge peuvent se succéder sans interruption, dès lors que le courant délivré lors de la phase de décharge ne s'annule pas. Ce mode de fonctionnement est appelé mode en conduction continue ou CCM, et il est adapté en termes de rendement quand le circuit utilisateur présente une charge quasi-permanente. En revanche, lorsque le courant de décharge dans l'inductance vient à s'annuler, un laps de temps peut s'écouler entre la fin de cette phase de décharge et le début de la phase de charge immédiatement consécutive. Ce mode de fonctionnement est appelé mode en conduction discontinue ou DCM, et ce mode est adapté, en termes de rendement, aux circuits utilisateurs présentant des charges très variables dans le temps.

Un *premier inconvénient* tient au fait que ces convertisseurs à découpage fonctionnent généralement de façon satisfaisante, avec un bon rendement, dès lors que l'appel de courant ne dépasse pas une valeur maximale, correspondant à un fonctionnement en conduction continue. En revanche, pour des appels de courant importants, le passage en conduction discontinue a pour effet de diminuer le rendement. Pour éviter cette situation, il est certes possible de diminuer la fréquence de commutation, pour allonger la durée des phases et charger l'inductance avec une énergie supérieure. La contrepartie est une augmentation importante du courant de crête en fin de charge, même lorsqu'il s'agit de délivrer des tensions de sortie relativement faibles. En d'autres termes, pour augmenter le courant à délivrer au circuit utilisateur, il est nécessaire d'augmenter dans des proportions très importantes le courant de crête admissible, avec des contraintes nouvelles sur le dimensionnement des composants afin qu'ils soient en mesure de supporter ce courant de crête accru.

Un *deuxième inconvénient* des convertisseurs à découpage connus est qu'ils sont en général mal adaptés à un fonctionnement "à la demande", c'est-à-dire un fonctionnement en tout-ou-rien pour des circuits qui ne sont utilisés que de façon sporadique, tels que les circuits d'émission/réception de télémétrie RF ou les circuits de délivrance d'un choc de défibrillation. En fonctionnement normal, ces circuits ne sont pas utilisés, donc pas alimentés, et le convertisseur qui les alimente est mis à l'arrêt ou mis en mode faible consommation. En revanche, lorsqu'ils doivent être utilisés, le convertisseur de mise en service doit délivrer très rapidement un courant très élevé (typiquement dans un intervalle de quelques fractions de milliseconde). Il s'agit donc de pouvoir fournir très vite la tension requise, ce qui est une situation très différente de celle consistant à réguler une tension lentement variable pour l'alimentation d'un circuit en fonctionnement permanent.

Un *troisième inconvénient* tient au fait que, pour mesurer le courant traversant l'inductance, il est nécessaire de placer en série avec celle-ci un élément résistif. La première conséquence en est l'introduction d'une perte de charge, qui vient obérer le rendement global. Au surplus, le vieillissement du composant sur une longue durée (typiquement sur dix ans, durée de vie habituelle d'un implant cardiaque) va entraîner une imprécision de plus en plus grande, au fil du temps, sur la mesure du courant de crête, qui pourra en réalité atteindre des niveaux insuffisants ou excessifs par rapport à la valeur prédéfinie. De ce fait, pour un courant de décharge maximal de l'ordre de 10 mA par exemple, l'inductance peut se trouver chargée avec un courant de pic allant jusqu'à 100 mA, induisant ici encore une perte de rendement.

Enfin, un *quatrième inconvénient* résulte du fait que le séquencement des phases de charge et de décharge est assuré par des circuits analogiques. L'imprécision de ces circuits, notamment la dispersion des valeurs des composants et les dérives des valeurs au cours de la vie de l'appareil, impose de prendre des marges de sécurité lors de la conception du convertisseur. Le convertisseur est notamment calculé sur la base d'une tension de batterie en fin de vie, de sorte que son fonctionnement ne sera pas optimal en début de vie, entraînant donc perte d'efficacité, consommation inutilement accrue et surdimensionnement des composants.

Compte tenu de ce qui vient d'être exposé, il existe ainsi un besoin pour une alimentation du type convertisseur à découpage qui soit particulièrement (mais de manière non limitative) adaptée à des implants médicaux actifs, palliant les différentes difficultés exposées ci-dessus et présentant notamment les caractéristiques suivantes :

- fonctionnement très réactif, autorisant des appels de courant brusques intervenant dans des intervalles de temps de quelques dizaines de microsecondes ;
- faible consommation intrinsèque, notamment avec un minimum de composants analogiques ;
- rendement de conversion élevé, avec faibles pertes dans les circuits de conversion et fonctionnement en mode CCM (à conduction continue) dans toute la mesure du possible ;
- intégration aisée sur une puce, avec un minimum de composants additionnels externes rapportés ;
- adaptation à une topologie quelconque *(buck, boost, buck-boost* ou *mixed)* en fonction du cahier des charges, sans qu'il soit besoin de redessiner le circuit du convertisseur ;
- capacité à travailler dans une large plage de tensions d'entrée (par exemple de 3,3 à 1,2V), par sa faculté à changer de topologie de manière dynamique ;

- capacité à fonctionner "à la demande", le convertisseur étant à l'arrêt la plupart du temps, mais lorsqu'il est mis en service (télémétrie RF, choc de défibrillation), réaction très rapide et délivrance d'un courant très élevé de façon pratiquement instantanée après activation.

L'invention propose à cet effet un dispositif médical actif d'un type connu comprenant : une batterie formant source d'alimentation du dispositif ; un circuit utilisateur à alimenter par la batterie à une tension nominale donnée ; et une alimentation à découpage inductive comprenant une entrée recevant une tension d'entrée continue non stabilisée produite par la batterie, et une sortie alimentant le circuit utilisateur par une tension de sortie stabilisée, différente de la tension d'entrée.

L'alimentation à découpage comprend une inductance de stockage d'énergie, un condensateur tampon, et un réseau de commutation apte à établir entre l'entrée, la sortie, l'inductance et le condensateur, au cours de cycles successifs et selon une topologie prédéfinie d'alimentation à découpage, au moins deux configurations alternatives avec : pendant la durée d'une phase de charge, une configuration d'accumulation d'énergie où l'inductance est chargée par un courant, délivré par la batterie, présentant une intensité croissante jusqu'à une valeur de crête ; et pendant la durée d'une phase de décharge, une configuration de restitution d'énergie où l'énergie accumulée dans l'inductance pendant la phase de charge est transférée au condensateur tampon, et du condensateur tampon au circuit utilisateur. Des moyens de pilotage du réseau de commutation commandent la fin des phases de charge et de décharge de manière à réguler la tension de sortie en fonction de la tension d'entrée par rapport à la tension nominale donnée.

De façon caractéristique de l'invention, les moyens de pilotage du réseau de commutation sont essentiellement dépourvus de moyens de mesure du courant traversant l'inductance, et ils comportent un convertisseur analogique-numérique propre à délivrer une valeur numérique représentative de la tension d'entrée. Ils comportent également des moyens prédicteurs, avec des moyens de calcul numérique aptes à évaluer *a priori* les durées des phases de charge et de décharge en fonction d'une pluralité de paramètres comprenant : la tension d'entrée, la tension de sortie, la tension nominale, la valeur de l'inductance, et une valeur prédéterminée du courant de crête traversant l'inductance lors de la phase de charge.

Dans une première forme de mise en oeuvre, les moyens prédicteurs comprennent une table de correspondance mémorisant des valeurs précalculées des durées des phases de charge et de décharge en fonction desdits paramètres.

Dans une deuxième forme de mis en oeuvre, les moyens prédicteurs comprennent des moyens de calcul dynamique des durées des phases de charge et de décharge en fonction desdits paramètres.

Dans ce dernier cas, les moyens de calcul dynamique des durées des phases de charge et de décharge comprennent avantageusement des moyens de détermination dynamique des durées des phases de charge et de décharge, notamment avec un automate fini activable conditionnellement par des requêtes de service.

Les moyens de pilotage peuvent en outre comprendre pour leur séquencement une horloge sélectivement activable, qui n'est activée que sur émission d'une requête de service, et désactivée après exécution de cette requête.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 est un schéma général d'une alimentation à convertisseur à découpage selon l'invention.

La Figure 2 illustre plus en détail le contrôleur numérique assurant le pilotage et le séquencement du convertisseur de la Figure 1.

On va maintenant décrire un exemple de réalisation du dispositif de l'invention.

L'invention peut notamment être appliquée aux dispositifs implantables tels que ceux des familles *Reply* et *Paradym* produits et commercialisés par ELA Médical, Montrouge, France. Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

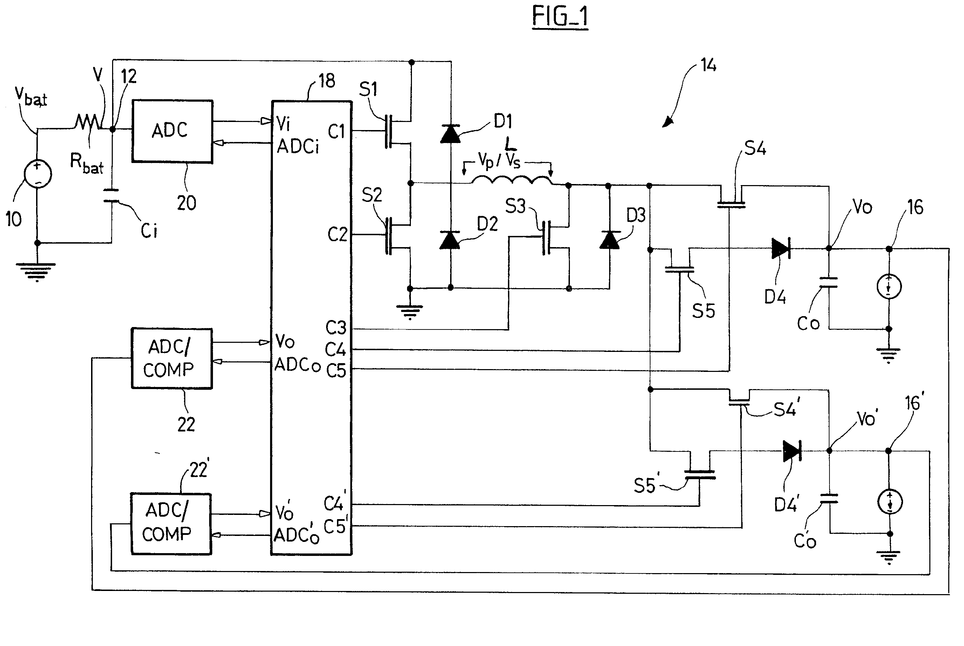

Sur la Figure 1, on a représenté un exemple d'alimentation pour dispositif médical implantable, avec un convertisseur à découpage pour la production et la régulation de la tension délivrée aux circuits utilisateurs.

L'exemple de la Figure 1 correspond à un convertisseur du type "SIMO" *(Single Input Multiple Output),* c'est-à-dire avec une unique source de tension continue en entrée (la batterie de l'implant) et plusieurs sorties d'alimentation régulées. Cette configuration permet d'alimenter des circuits nécessitant des tensions d'alimentation différentes, ou encore des circuits dont certains sont utilisés de façon continue (circuits de détection et de stimulation par exemple), tandis que d'autres ne le sont que ponctuellement, dans un mode "à la demande" (circuit émetteur/récepteur de télémétrie, ou circuit de délivrance d'un choc de défibrillation par exemple), avec au surplus un appel de courant important lors de ces utilisations ponctuelles.

Sur la Figure 1 on a illustré une configuration à deux étages de conversion correspondant à deux sorties distinctes, mais cet exemple n'est bien entendu aucunement limitatif. De la même façon, les différentes topologies de convertisseurs envisageables, bien connues de l'homme du métier, ne seront pas exposées en détail *(buck* pour réduire la tension d'entrée, *boost* pour l'augmenter, *mixed* ou *buck-boost* pour pouvoir aussi bien l'augmenter que la réduire, avec inversion de polarité dans un cas, sans inversion dans l'autre. La sélection de l'une ou l'autre topologie peut se faire sans modification du circuit (à l'exception de l'inversion de la diode de sortie pour la topologie *buck-boost),* par une simple modification des diverses commutations lors des phases de charge et de décharge de l'inductance du convertisseur.

L'alimentation comprend une batterie 10 de tension nominale $V_{BAT}$, valeur décroissant régulièrement au fil des années sur toute la durée de vie de la batterie. Compte tenu de son impédance interne $R_{BAT}$, la batterie délivre une tension $V_I$ appliquée à l'entrée 12 du convertisseur 14 et qui est fonction de l'appel de courant par les circuits à alimenter. Le convertisseur 14 délivre sur ses deux sorties 16, 16' deux tensions de sortie régulées $V_O$, $V_O'$ pour l'alimentation de circuits utilisateurs respectifs du dispositif.

Le convertisseur 14 est pourvu de condensateurs tampons d'entrée $C_I$ et de sortie $C_O$, $C'_O$ permettant d'absorber les variations à court terme des tensions d'entrée et de sortie. La capacité de ces condensateurs est choisie suffisante pour que l'on puisse considérer que, sur la durée d'une phase de charge ou d'une phase de décharge (voir plus bas), les tensions d'entrée $V_I$ et de sortie $V_O$, $V'_O$ sont sensiblement constantes.

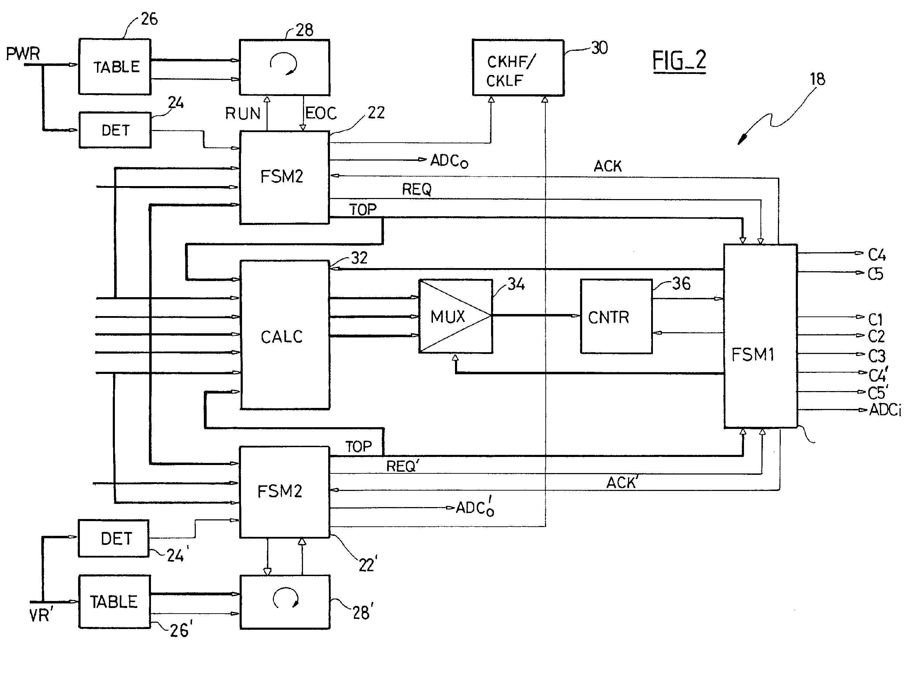

Le convertisseur 14 comprend une inductance L soumise alternativement à des phases de charge et de décharge par l'intermédiaire de commutateurs électroniques S1, S2, S3, S4, S5 (et S4', S5' pour le second étage) et de diodes D1, D2, D3 et D4 (et D4' pour le second étage). Les interrupteurs sont commandés par des signaux respectifs C1, C2, C3, C4, C5 (et C4', C5') produits par un circuit numérique 18 dont la structure détaillée et le fonctionnement seront exposés en détail par la suite, en référence à la Figure 2.

Le circuit de pilotage numérique 18 reçoit en entrée une information numérique représentative de la tension d'entrée $V_I$ après que celle-ci ait été numérisée par un convertisseur analogique/numérique 20 activé à la demande par un signal $ADC_i$. Ce convertisseur 20 présente une résolution suffisante pour pouvoir suivre les variations à court terme et à long terme de la tension $V_I$ appliquée en entrée du convertisseur, et il constitue de ce fait un organe essentiel du convertisseur de l'invention.

Le circuit 18 reçoit également des informations sur les tensions de sortie $V_O$ et $V_O'$ par l'intermédiaire de circuits respectifs 22, 22' qui peuvent être soit des convertisseurs analogique/numérique activables sélectivement par des signaux $ADC_O$ et $ADC_O'$, soit de simples comparateurs. Ce dernier cas peut en particulier s'appliquer dans une configuration de régulation où l'on est certain que la tension de sortie $V_O$ à réguler est proche de sa valeur nominale $V_{ref}$ il suffit alors en effet de déterminer si cette tension de sortie $V_O$ est ou non comprise dans une plage $V_{ref} \pm V_A/2$, *VA* étant le pas de régulation de la tension. L'utilisation de comparateurs au lieu de convertisseurs permet notamment de simplifier le circuit et d'en diminuer la consommation.

On va maintenant décrire un cycle de fonctionnement du convertisseur, dans un exemple de fonctionnement en topologie *mixed* (donc sans inversion de polarité) sur la première sortie 16 (le fonctionnement de la sortie 16' du second étage est comparable, et ne sera pas décrit).

Comme on le sait, le fonctionnement cyclique d'un convertisseur à découpage implique une phase de charge (ou phase primaire) et une phase de décharge (ou phase secondaire). Plus précisément :

1°) Phase de charge (phase primaire) : S1, S3, S5 sont fermés, S2, S4 sont ouverts. L'inductance L, initialement déchargée, est montée entre l'entrée 12 et la masse, de sorte que la tension primaire $V_P$ à ses bornes est égale à $V_I$. Cette inductance est chargée pendant une durée donnée $T_P$, jusqu'à ce que le courant atteigne la valeur maximale prescrite, valeur qui sera appelée courant de crête $I_K$. Au premier ordre, si l'on néglige tout phénomène résistif en série avec l'inductance, et si l'on considère que les condensateurs d'entrée et de sortie $C_I$ et $C_O$ présentent une capacité suffisante pour que les tensions $V_I$ et $V_O$ puissent être considérées constantes pendant toute la durée du cycle, on a : $T_P.V_P = L.I_K$.

2°) À la fin de la phase de charge, lors d'une période de transition de très courte durée, les commutateurs S1 et S3 sont ouverts de manière à éviter, juste avant la fermeture de S2 et S4 (à l'étape suivante), l'apparition d'un chemin à très faible impédance entre l'entrée et la masse ; lors de cette transition, l'inductance commence à se décharger dans les diodes D2 et D4.

3°) Phase de décharge (phase secondaire), première partie : S2, S4, S5 sont fermés, S1, S3 sont ouverts. L'inductance, qui est alors connectée entre la sortie 16 et la masse de sorte que la tension secondaire $V_S$ à ses bornes est égale à $V_O$, est déchargée pendant une durée donnée $T_{S1}$, jusqu'à ce que le courant atteigne une fraction fixe $p$ (par exemple 70 %) de la valeur initiale de crête $I_K$. Avec comme précédemment une approximation au premier ordre, on aura: $T_{S1}. V_S = P.LI_K$.

4°) Phase secondaire, deuxième partie : S4 est alors ouvert, ce qui place la diode de roue libre D4 dans le chemin de décharge de l'inductance. Celle-ci reste toujours reliée entre la sortie 16 et la masse, mais une inversion du courant de décharge est évitée. La décharge se poursuit pendant une durée $T_{S2}$ jusqu'à un courant nul. La limite supérieure de $T_{S2}$ est donnée par $T_{S2}$. $V_S = (1 - p) L.I_K$.

5°) Fin de la phase de décharge : le commutateur S5 est ouvert, ce qui met à la masse l'inductance L via S2 et S3 et évite toute suroscillation.

L'idée de base de l'invention consiste à calculer *a priori* la durée de la phase primaire de charge $T_P$ et la durée de la phase secondaire de décharge $T_S$ ($T_S = T_{S1} + T_{S2}$) en fonction d'informations d'entrée et de sortie mesurées, et du courant de crête à atteindre. Cette manière de procéder s'oppose à l'approche classique consistant à mesurer le courant traversant l'inductance, à en suivre les variations tout au long du cycle de charge, et à interrompre la charge lorsque ce courant atteint, la valeur prédéfinie du courant de crête, les durées des phases de charge et de décharge s'établissant alors *a posteriori* comme conséquence de cette manière de contrôler la charge et la décharge.

Les relations de base donnant $T_P$ et $T_S$ sont :

$$T_P = -\frac{L}{R_P}\log\left(1 - \frac{R_P I_K}{V_P}\right), \quad \text{et} \quad T_S = +\frac{L}{R_S}\log\left(1 + \frac{R_S I_K}{V_S}\right),$$

$R_P$ et $R_S$ étant les valeurs des composantes résistives en série avec l'inductance $L$ lors des phases respectives de charge et de décharge.

On a indiqué plus haut que ces relations pouvaient être approximées au premier ordre sous la forme simplifiée :

$$T_P = \frac{L I_K}{V_P} \quad \text{et} \quad T_S = \frac{L I_K}{V_S},$$

$V_P$ et $V_S$ étant choisis en fonction de la topologie de convertisseur retenue, conformément au tableau suivant :

|  | *Buck* | *Boost* | *Mixed* | *Buck-Boost* |
|---|---|---|---|---|
| $V_P$ | $V_I - V_O$ | $V_I$ | $V_I$ | $V_I$ |
| $V_S$ | $V_O$ | $V_O - V_I$ | $V_O$ | $- V_O$ |

(on notera que dans une topologie *buck-boost* la tension $V_O$ est négative). Comme on peut le constater, les durées des phases primaire et secondaire sont essentiellement fonction des tensions d'entrée et de sortie, ainsi que de l'inductance $L$ et d'un courant de crête $I_K$ prédéterminé, qui dépendra de la topologie.

Les deux seuls paramètres susceptibles de varier au cours du temps sont la tension d'entrée $V_I$ et la tension de sortie $V_O$. Toutefois, comme indiqué plus haut, si l'on est sûr que le convertisseur ne fonctionnera que lorsque la tension de sortie $V_O$ est proche de sa valeur nominale $V_{ref}$ (c'est-à-dire sera toujours dans la plage $V_{ref} \pm V_A/2$), la tension de sortie peut être considérée comme constante au cours du temps. Il est alors possible de procéder d'une manière simple, en calculant au préalable les valeurs des durées primaire et secondaire pour chaque valeur de la tension d'entrée, et en mémorisant ces durées dans une table de correspondance (avec une table pour chaque topologie), ou même dans un simple registre si l'on se contente de topologies simplifiées, comme en mode *buck* ou *mixed* avec une valeur $T_S$ constante.

L'autre approche possible est un calcul direct, en temps réel, des durées $T_P$ et $T_S$, calcul exécuté par des circuits appropriés de pilotage numérique en fonction des valeurs $V_I$ et $V_O$ mesurées instantanées.

On va maintenant décrire un exemple d'implémentation du circuit de pilotage numérique 18, en référence à la Figure 2.

Ce circuit comporte trois automates finis FSM *(Finite State Machine)* référencés 20, 22 et 22'.

L'automate 20 est un contrôleur de commutation, qui pilote les divers commutateurs S1, S2, ..., S5 décrits plus haut à propos de la Figure 1. Les automates 22 et 22' sont des moniteurs qui surveillent les tensions de sortie $V_O$ et $V_O'$ sur les sorties respectives 16 et 16'. Le fonctionnement de l'automate 22' qui est destiné au contrôle du second étage du convertisseur (sortie 16'), ne sera pas décrit en détail, non plus que les autres circuits de commande de cet étage, qui sont identiques à ceux du premier étage (sortie 16).

Lorsque le moniteur 22 détecte que la tension de sortie $V_O$ est descendue au-dessous de sa valeur nominale, il délivre au contrôleur de commutation 20 une requête de service REQ. Si le contrôleur 20 n'est pas déjà actif du fait d'une requête provenant de l'autre moniteur 22', il initie un cycle de charge/décharge pour satisfaire cette requête. Une fois ce cycle achevé, l'automate 20 envoie au moniteur 22 un accusé de réception ACK et se met en attente d'une autre requête. Le moniteur 20, quant à lui, continue à surveiller la tension de sortie $V_O$.

Les circuits associés aux automates finis 20, 22 sont conçus de manière à réduire la consommation du circuit de pilotage 18, car il s'agit là d'un aspect essentiel des convertisseurs à découpage, tout particulièrement ceux qui sont utilisés au sein d'implants médicaux.

Une première mesure d'économie d'énergie consiste à optimiser l'utilisation de l'horloge système.

L'horloge système est une horloge CKHF à haute fréquence, de l'ordre de quelques mégahertz ou plus, donc avec une période $T_C$ compatible avec les algorithmes de calcul des durées $T_P$ et $T_S$.

Dans la mesure où la consommation d'un système numérique est approximativement proportionnelle à sa fréquence d'horloge, il est important d'arrêter cette horloge lorsque le système n'est pas en service, par exemple lorsque les tensions de sortie sont toutes au-dessus de leurs valeurs nominales et que le système est simplement utilisé pour surveiller le moment où ces tensions baisseront au-dessous de ces seuils. L'horloge CKHF ne sera activée que lorsque l'un des organes du régulateur, typiquement le moniteur 22 ou 22', délivrera une requête de service, et sera désactivée dès que la requête aura été traitée par le contrôleur de commutation 20.

Cette manière de procéder implique que ces blocs 22, 22' et les circuits qui leur sont directement associés puissent fonctionner sans l'horloge système CKHF.

Une première technique consiste à utiliser pour ces blocs des circuits analogiques, par exemple des comparateurs, qui seront alimentés de façon continue. Cette manière de procéder est simple et sûre, mais elle implique une consommation permanente d'au moins quelques microampères. Une autre technique, préférentielle, consiste à se passer presque complètement de tout élément analogique, en utilisant un circuit numérique séquencé par une deuxième horloge, ci-après désignée CKLF, qui sera active en permanence mais dont la fréquence sera très inférieure à celle de l'horloge CKHF, par exemple une fréquence à 32 kHz. Dans la mesure où, avec une telle valeur, la durée entre deux cycles successifs de charge/ décharge du convertisseur peut être considérée comme longue par rapport à la période de l'horloge basse fréquence CKLF, il est possible d'assurer un suivi de la tension de sortie tout en minimisant la consommation requise pour cette fonction.

Si l'on peut estimer les valeurs de courant et les instants où des appels de courant sont susceptibles de survenir (par exemple, on sait à l'avance qu'un choc de défibrillation va être délivré, ou qu'une fonction de télémétrie va être activée), il est possible d'implémenter un contrôle totalement numérique de la tension de sortie, avec un temporisateur qui met hors service l'horloge haute fréquence CKHF pendant une durée longue, par exemple une milliseconde. Dès lors que l'on peut être sûr que sur une telle durée, compte tenu de la capacité du condensateur de sortie $C_O$, la tension de sortie $V_O$ ne chutera pas de plus d'une valeur prédéterminée, par exemple pas de plus de 5 mV, le convertisseur pourra immédiatement compenser cette chute de tension dès le prochain cycle de charge/décharge.

Si l'on revient à la Figure 2, le circuit de pilotage 18 reçoit en entrée un bus numérique PWR représentatif de la consommation courante du dispositif. Cette valeur est stockée dans un registre d'un détecteur 24, qui détecte toute variation de ce signal afin de pouvoir réveiller le moniteur 22 si celui-ci est dans un état de sommeil de l'automate fini.

Une table 26 mémorise, pour chaque niveau de consommation PWR un certain nombre d'informations telles que :

- intervalle entre deux contrôles du niveau de la tension de sortie $V_O$;
- courant de crête $I_K$ en fonction du courant appelé (une augmentation du courant de crête permet d'augmenter la période entre deux détections successives, et inversement) ;
- sélection de l'horloge à utiliser (pour des niveaux de consommation élevés, l'horloge rapide peut être nécessaire afin d'augmenter la réactivité du convertisseur).

Un temporisateur 28 est utilisé pour déterminer les intervalles auxquels seront détectées les variations de la tension de sortie $V_O$. Ce temporisateur est activé par le moniteur 22 de mesure de la tension $V_O$ lorsque ce moniteur se met en sommeil. Le moniteur en sommeil recevra plus tard un signal de réveil EOC lorsque le temporisateur 28 aura fini son comptage. Le fonctionnement du moniteur 22, qui est réalisé sous forme d'un automate fini FSM, est le suivant.

Tout d'abord, le moniteur produit un signal $ADC_O$ d'activation du convertisseur de mesure de la tension de sortie $V_O$ (convertisseur qui peut être remplacé, comme indiqué plus haut, par un simple comparateur).

Si la tension de sortie est supérieure à la valeur nominale $V_{ref}$, le moniteur active le temporisateur 28 (signal RUN). Le moniteur se met ou non en sommeil selon l'horloge utilisée par le compteur 28. Si celui-ci fonctionne avec l'horloge système CKHF, le moniteur 22 se met en boucle d'attente, mais continue à activer le gestionnaire d'horloge 30 pour que celui-ci maintienne l'horloge système haute fréquence CKHF active. Dans le cas contraire, le moniteur 22 peut informer le gestionnaire d'horloge 30 de la possibilité de désactiver l'horloge système CKHF (si l'autre moniteur 22' ne délivre pas de requête en sens contraire, bien entendu). Le moniteur 22 permet également de choisir la topologie de commutation, et cette information TOP est transmise à la fois à l'automate 20 de pilotage des commutateurs et au calculateur 32 des durées des phases de charge et de décharge.

Le circuit de pilotage des commutateurs 20 est réalisé, comme indiqué plus haut, sous forme d'un automate fini FSM qui fonctionne en boucle, dans l'attente d'une requête de service REQ en provenance de l'un des moniteurs 22 ou 22'.

Sur réception d'une telle requête, une mesure de la tension d'entrée est effectuée (activation du convertisseur 20 de la Figure 1 par un signal d'activation $ADC_I$). La valeur mesurée est appliquée au calculateur 32 qui détermine les durées $T_P$ et $T_S$ (plus précisément, $T_P$, $T_S$, et $T_{S2}$) sous forme d'un multiple de la période $T_C$ de l'horloge CKHF. Les valeurs calculées par le circuit numérique 32 sont utilisées, via le multiplexeur 34 et le compteur 36, pour réaliser les différentes commutations exposées plus haut, en fonction des durées calculées et de la topologie sélectionnée. Comme on l'a indiqué plus haut, le calculateur 32 peut déterminer les diverses durées $T_P$, $T_{S1}$ et $T_{S2}$ soit à partir d'une table de correspondances, soit en exécutant directement les calculs de ces valeurs, en temps réel. Les paramètres à prendre en compte pour déterminer $T_P$, $T_S$ et $T_{S2}$ sont:

- la tension d'entrée $V_I$;
- la tension de sortie $V_O$;
- la tension nominale $V_{ref}$;
- la topologie utilisée;
- l'inductance $L$ ;
- le courant de crête $I_K$ (avec des valeurs éventuellement différentes en fonction de la topologie) ;
- le pourcentage p définissant le début de la deuxième partie de la phase de décharge $T_{S2}$ où la diode de roue libre est insérée dans le trajet de décharge pour éviter l'inversion de courant ;
- la résistance $R_L$ de l'inductance (si l'on veut utiliser des modèles de calculs plus précis).

Certains de ces paramètres sont le résultat d'une conversion analogique/numérique ($V_I$, $V_O$), d'autres sont déterminés par un autre bloc (choix de la topologie), d'autres sont stockés en mémoire ou dans un jeu de registre ($V_{ref}$, $I_K$, $L$, $R$, $p$).

Dans la technique consistant à utiliser une table de correspondance, les durées $T_P$, $T_{S1}$ et $T_{S2}$ sont calculées au préalable et mémorisées dans une table ou dans des registres. Dans la mesure où le temps de calcul n'est pas un obstacle, cette technique permet en particulier d'utiliser des relations mathématiques complexes, prenant en compte un grand nombre de paramètres et/ou des fonctions non linéaires.

En revanche, dans le cas d'un calcul direct en temps réel il est nécessaire d'utiliser des modèles simplifiés pour calculer les divers paramètres. En pratique, cette limitation n'est pas trop gênante, et présente l'avantage de pouvoir modifier à tout moment les paramètres du calcul, par exemple pour adapter le courant de crête $I_K$ en fonction de la topologie sélectionnée ou du niveau de consommation. De même, la tension nominale $V_{ref}$ et la valeur de l'inductance peuvent être adaptées dans le temps pour prendre en compte des modifications du système tel que le vieillissement ou d'autres modifications qui requièrent une modification de la tension d'alimentation.

Pour le calcul de la durée de la phase primaire de charge $T_P$, la relation à utiliser est :

$$T_P = \frac{L\,I_K}{V_P} \ ,$$

qui peut être réécrite après discrétisation sous la forme :

$$t_P = \frac{L\,I_{KO}}{T_C V_A} \cdot \frac{k}{v_P} \ ,$$

avec $T_P = T_C t_P$, $IK = I_{KO}k$, $V_P = V_A v_P$ ,
$t_p$, $k$ et $v_P$ étant des entiers,
$I_{KO}$ étant une valeur minimale programmable du courant de crête, et

$v_P$ étant, sous forme entière, le résultat de la conversion de tension analogique/numérique.

Le calcul de la durée de la phase de décharge $T_S$ est réalisé de façon comparable, avec $v_S$ au lieu de $v_P$. Les durées des deux phases $T_{S1}$ et $T_{S2}$ sont données par $T_{S1} = p.T_S$ et $T_{S2} = T_S - T_{S1}$. On peut choisir pour $p$ une valeur fixe, par exemple $p = 0,75$, pour simplifier le circuit.

On notera que, dans la mesure où $T_P$, $T_{S1}$ et $T_{S2}$ sont des données dont on n'a pas besoin simultanément, mais successivement, leur calcul n'a pas besoin d'être effectué en parallèle et il peut être sérialisé et effectué au moyen des mêmes circuits. En revanche, si l'on utilise une table de correspondance, une table doit être prévue pour chacune de ces données.

**Revendications**

1. Un dispositif médical actif, comprenant :

    - une batterie (10) formant source d'alimentation du dispositif;
    - un circuit utilisateur à alimenter par la batterie à une tension nominale donnée $(V_{ref})$; et
    - une alimentation à découpage inductive (14) comprenant une entrée (12) recevant une tension d'entrée continue non stabilisée $(V_I)$ produite par la batterie, et une sortie (16) alimentant le circuit utilisateur par une tension de sortie stabilisée $(V_O)$, différente de la tension d'entrée, l'alimentation à découpage comprenant :
    - une inductance (L) de stockage d'énergie;
    - un condensateur tampon de sortie $(C_O)$;
    - un réseau de commutation (S1 ... S5, D1 ... D4), apte à établir entre l'entrée, la sortie, l'inductance et le condensateur tampon, au cours de cycles successifs et selon une topologie prédéfinie d'alimentation à découpage, au moins deux configurations alternatives, avec :

        · pendant la durée d'une phase de charge, une configuration d'accumulation d'énergie où l'inductance est chargée par un courant délivré par la batterie, ce courant présentant une intensité croissante jusqu'à une valeur de crête, et
        · pendant la durée d'une phase de décharge, une configuration de restitution d'énergie où l'énergie accumulée dans l'inductance pendant la phase de charge est transférée au condensateur tampon, et du condensateur tampon au circuit utilisateur ; et

    - des moyens (18, 20, 22) de pilotage du réseau de commutation, aptes à commander la fin des phases de charge et de décharge de manière à réguler la tension de sortie en fonction de la tension d'entrée par rapport à la tension nominale donnée,

    dispositif **caractérisé en ce que** lesdits moyens de pilotage du réseau de commutation :

    - sont dépourvus de moyens de mesure du courant traversant l'inductance,
    - comportent un convertisseur analogique-numérique (20) propre à délivrer une valeur numérique représentative de la tension d'entrée $(V_I)$, et
    - comportent des moyens prédicteurs, comprenant des moyens de calcul numérique aptes à évaluer a *priori* les durées des phases de charge et de décharge en fonction d'une pluralité de paramètres comprenant : la tension d'entrée $(V_I)$, la tension de sortie $(V_O)$, la tension nominale $(V_{ref})$, la valeur de l'inductance $(L)$, et une valeur prédéterminée du courant de crête $(I_K)$ traversant l'inductance lors de la phase de charge.

2. Le dispositif de la revendication 1, dans lequel les moyens prédicteurs comprennent une table de correspondance mémorisant des valeurs précalculées des durées des phases de charge et de décharge en fonction desdits paramètres.

3. Le dispositif de la revendication 1, dans lequel les moyens prédicteurs comprennent des moyens de calcul dynamique desdites durées des phases de charge et de décharge en fonction desdits paramètres.

4. Le dispositif de la revendication 3, dans lequel les moyens de calcul dynamique des durées des phases de charge et de décharge comprennent des moyens de détermination dynamique desdites durées.

5. Le dispositif de la revendication 4, dans lequel les moyens de détermination dynamique des durées des phases de charge et de décharge comprennent un automate fini activable conditionnellement par des requêtes de service.

**6.** Le dispositif de la revendication 5, dans lequel les moyens de pilotage comprennent pour leur séquencement une horloge sélectivement activable, cette horloge n'étant activée que sur émission d'une requête de service, et désactivée après exécution de cette requête.

**Claims**

**1.** An active medical device comprising:

- a battery (10) forming a power source of the device;
- a user circuit to be powered by the battery at a given nominal voltage ($V_{ref}$); and
- an inductive chopping power supply (14) comprising an input (12) receiving an unstabilized direct-current input voltage ($V_I$) produced by the battery, and an output (16) powering the user circuit with a stabilized output voltage ($V_O$), different from the input voltage,

the chopping power supply comprising:

- an energy-storage inductor (L);
- an output buffer capacitor ($C_O$);
- a switching network (S1 ... S5, D1 ... D4) capable of establishing between the input, the output, the inductor and the buffer capacitor, during successive cycles and according to a predefined chopping power supply topology, at least two alternative configurations, with:

    • during the period of a charging phase, a power-accumulation configuration in which the inductor is charged by a current delivered by the battery, this current having an intensity that increases up to a peak value, and
    • during the period of a discharging phase, a configuration of restoring power in which the power accumulated in the inductor during the charging phase is transferred to the buffer capacitor, and from the buffer capacitor to the user circuit; and

- means (18, 20, 22) for controlling the switching network that are capable of commanding the end of the charging and discharging phases so as to regulate the output voltage as a function of the input voltage relative to the given nominal voltage,

the device being **characterized in that** the said means for controlling the switching network:

- are bereft of means for measuring the current passing through the inductor,
- comprise an analogue-digital converter (20) suitable for delivering a digital value representative of the input voltage ($V_I$), and
- comprising predicting means, comprising digital computing means capable of evaluating *a priori* the durations of the charging and discharging phases as a function of a plurality of parameters comprising: the input voltage ($V_I$), the output voltage ($V_O$), the nominal voltage *($V_{ref}$)*, the value of the inductor (L), and a predetermined value of the peak current ($I_K$) passing through the inductor during the charging phase.

**2.** The device of Claim 1, in which the predicting means comprise a mapping table storing precomputed values of the durations of the charging and discharging phases as a function of the said parameters.

**3.** The device of Claim 1, in which the predicting means comprise computing means for dynamically computing the said durations of the charging and discharging phases as a function of the said parameters.

**4.** The device of Claim 3, in which the computing means for dynamically computing the durations of the charging and discharging phases comprise determination means for dynamically determining the said durations.

**5.** The device of Claim 4, in which the determination means for dynamically determining the durations of the charging and discharging phases comprise a finite-state machine that can be activated conditionally by service requests.

**6.** The device of Claim 5, in which the control means comprise for their sequencing a clock that can be activated selectively, this clock being activated only on transmission of a service request, and deactivated after execution of this request.

**Patentansprüche**

1. Aktive medizinische Vorrichtung, die enthält:

   - eine Batterie (10), die eine Stromquelle der Vorrichtung bildet;
   - eine von der Batterie mit einer gegebenen Nennspannung ($V_{ref}$) zu versorgende Benutzerschaltung; und
   - ein induktives Schaltnetzteil (14) mit einem Eingang (12), der eine von der Batterie erzeugte, unstabilisierte Eingangsgleichspannung ($V_I$) empfängt, und mit einem Ausgang (16), der die Benutzerschaltung mit einer stabilisierten Ausgangsspannung ($V_o$) versorgt, die sich von der Eingangsspannung unterscheidet,

   wobei das Schaltnetzteil enthält:

   - eine Energiespeicher-Drosselspule (L);
   - einen Ausgangs-Pufferkondensator ($C_o$);
   - ein Umschaltnetzwerk (S1 ... S5, D1 ... D4), das zwischen dem Eingang, dem Ausgang, der Drosselspule und dem Pufferkondensator während aufeinanderfolgender Zyklen und gemäß einer vordefinierten Topologie des Schaltnetzteils mindestens zwei alternative Konfigurationen aufbauen kann, mit:

     • während der Dauer einer Ladephase einer Energiespeicher-Konfiguration, in der die Drosselspule mit einem von der Batterie gelieferten Strom geladen wird, wobei dieser Strom eine bis zu einem Spitzenwert zunehmende Stärke aufweist, und
     • während der Dauer einer Entladephase eine Energiewiederherstellungs-Konfiguration, in der die in der Drosselspule während der Ladephase gespeicherte Energie zum Pufferkondensator und vom Pufferkondensator zur Benutzerschaltung übertragen wird; und

   - Einrichtungen (18, 20, 22) zur Steuerung des Umschaltnetzwerks, die fähig sind, das Ende der Lade- und Entladephasen so zu steuern, dass die Ausgangsspannung in Abhängigkeit von der Eingangsspannung bezüglich der gegebenen Nennspannung geregelt wird,

   Vorrichtung, die **dadurch gekennzeichnet ist, dass** die Steuereinrichtungen des Umschaltnetzwerks:

   - keine Einrichtungen zum Messen des die Drosselspule durchfließenden Stroms aufweisen,
   - einen Analog-Digital-Wandler (20) aufweisen, der einen für die Eingangsspannung ($V_I$) repräsentativen digitalen Wert liefern kann, und
   - prädiktive Einrichtungen aufweisen, die Einrichtungen zur digitalen Berechnung enthalten, die a priori die Dauern der Lade- und Entladephasen in Abhängigkeit von mehreren Parametern ermitteln können, die enthalten: die Eingangsspannung ($V_I$), die Ausgangsspannung ($V_o$), die Nennspannung ($V_{ref}$), den Wert der Drosselspule (L), und einen vorbestimmten Wert des Spitzenstroms ($I_K$), der die Drosselspule in der Ladephase durchfließt.

2. Vorrichtung nach Anspruch 1, bei der die prädiktiven Einrichtungen eine Entsprechungstabelle enthalten, die vorberechnete Werte der Dauern der Lade- und Entladephasen in Abhängigkeit von den Parametern speichert.

3. Vorrichtung nach Anspruch 1, bei der die prädiktiven Einrichtungen Einrichtungen zur dynamischen Berechnung der Dauern der Lade- und Entladephasen in Abhängigkeit von den Parametern enthalten.

4. Vorrichtung nach Anspruch 3, bei der die Einrichtungen zur dynamischen Berechnung der Dauern der Lade- und Entladephasen Einrichtungen zur dynamischen Bestimmung der Dauern enthalten.

5. Vorrichtung nach Anspruch 4, bei der die Einrichtungen zur dynamischen Bestimmung der Dauern der Lade- und Entladephasen einen endlichen Automaten enthalten, der fallweise durch Dienstanforderungen aktivierbar ist.

6. Vorrichtung nach Anspruch 5, bei der die Steuereinrichtungen für ihre Folgesteuerung einen selektiv aktivierbaren Taktgeber enthalten, wobei dieser Taktgeber nur beim Senden einer Dienstanforderung aktiviert und nach Ausführung dieser Anforderung deaktiviert wird.

# FIG_1

FIG_2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5769877 A
- EP 0719570 A1
- EP 1647303 A1
- DE 102006008495 A1
- US 20080077010 A1
- US 20090043244 A1
- US 200400892982 A1